# EUROPEAN PATENT SPECIFICATION

(11) **EP 3 660 037 B1**
(45) Date of publication and mention of the grant of the patent: **13.04.2022**
(21) Application number: 19212382.6
(22) Date of filing: 29.11.2019
(51) Int. Cl.: C07K 14/435

(54) **FUSION POLYPEPTIDE AND METHOD FOR PRODUCING FUSION POLYPEPTIDE**
FUSIONSPOLYPEPTID UND VERFAHREN ZUR HERSTELLUNG VON FUSIONSPOLYPEPTID
POLYPEPTIDE DE FUSION ET PROCÉDÉ DE PRODUCTION D'UN POLYPEPTIDE DE FUSION

(30) Priority: 29.11.2018 JP 2018223373
(43) Date of publication of application: 03.06.2020
(73) Proprietor: SYSMEX CORPORATION, Kobe-shi Hyogo 651-0073 (JP)
(72) Inventor: BAJAJ, Reema, Kobe-shi, Hyogo, 651-0073 (JP); FUKUNAGA, Atsushi, Kobe-shi, Hyogo, 651-0073 (JP)
(74) Representative: De Clercq & Partners

(56) References cited:
- DATABASE UniProt [Online] 31 January 2018 (2018-01-31), "SubName: Full=Uncharacterized protein {ECO:0000313|EMBL:PBL01103.1};", XP002797613, retrieved from EBI accession no. UNIPROT:A0A2H3EBQ4 Database accession no. A0A2H3EBQ4
- DATABASE UniProt [Online] 29 October 2014 (2014-10-29), "SubName: Full=AGAP003349-PB-like protein {ECO:0000313|EMBL:KFB42119.1};", XP002797614, retrieved from EBI accession no. UNIPROT:A0A084VVX5 Database accession no. A0A084VVX5
- DATABASE UniProt [Online] 11 November 2015 (2015-11-11), "SubName: Full=Hpt domain {ECO:0000313|EMBL:CUA83995.1};", XP002797615, retrieved from EBI accession no. UNIPROT:A0A0K6GZJ0 Database accession no. A0A0K6GZJ0
- DATABASE UniProt [Online] 14 May 2014 (2014-05-14), "RecName: Full=MFS domain-containing protein {ECO:0000259|PROSITE:PS50850};", XP002797616, retrieved from EBI accession no. UNIPROT:X1WJM7 Database accession no. X1WJM7
- STUBENRAUCH K ET AL: "Purification of a viral coat protein by an engineered polyionic sequence", JOURNAL OF CHROMATOGRAPHY B, ELSEVIER SCIENCE PUBLISHERS, NL, vol. 737, no. 1-2, 1 January 2000 (2000-01-01), pages 77-84, XP004184256, ISSN: 0378-4347, DOI: 10.1016/S0378-4347(99)00392-8
- MARK Q. NIEDERAUER ET AL: "Characterization and Polyelectrolyte Precipitation of .beta.-Galactosidase Containing Genetic Fusions of Charged Polypeptides", BIOTECHNOLOGY PROGRESS, vol. 10, no. 3, 1 May 1994 (1994-05-01), pages 237-245, XP55667976, ISSN: 8756-7938, DOI: 10.1021/bp00027a001
- FISCHER B ET AL: "Isolation, renaturation, and formation of disulfide bonds of eukaryotic proteins expressed in Escherichia coli as inclusion bodies", BIOTECHNOLOGY AND BIOENGINEERING,, vol. 41, no. 1, 5 January 1993 (1993-01-05), pages 3-13, XP000605146, ISSN: 0006-3592, DOI: 10.1002/BIT.260410103
- MISICKA A ET AL: "Optimization of disulfide bond formation", POLISH JOURNAL OF CHEMISTRY, POLSKIE TOWARZYSTWO CHEMICZNE, POLISH CHEMICAL SOCIETY, PL, vol. 68, 1 January 1994 (1994-01-01), page 893, XP009516278, ISSN: 0137-5083
- GLEITER S ET AL: "Disulfide bond isomerization in prokaryotes", BIOCHIMICA ET BIOPHYSICA ACTA. MOLECULAR CELL RESEARCH, ELSEVIER SCIENCE PUBLISHERS, AMSTERDAM, NL, vol. 1783, no. 4, 1 April 2008 (2008-04-01), pages 530-534, XP022588094, ISSN: 0167-4889 [retrieved on 2008-02-23]

### SEQUENCE LISTING

<110> SYSMEX CORPORATION
<120> Fusion polypeptide and method for producing fusion polypeptide
<130> SYSM-145-EP
<150> JP2018-332273
   <151> 2018-11-29
<160> 16
<170> PatentIn version 3.5
<210> 1
   <211> 7
   <212> PRT
   <213> Artificial Sequence
<220>
   <223> Tag sequence
<400> 1
   Asp Asp Asp Cys Asp Asp Asp
   1               5
<210> 2
   <211> 9
   <212> PRT
   <213> Artificial Sequence
<220>
   <223> Tag sequence
<400> 2
   Asp Asp Asp Asp Asp Asp Cys Asp Asp
   1               5
<210> 3
   <211> 9
   <212> PRT
   <213> Artificial Sequence
<220>
   <223> Tag sequence
<400> 3
   Asp Asp Asp Asp Cys Asp Asp Asp Asp
   1               5
<210> 4
   <211> 7
   <212> PRT
   <213> Artificial Sequence
<220>
   <223> Tag sequence
<400> 4
   Asp Asp Asp Asp Cys Asp Asp
   1               5
<210> 5
   <211> 7
   <212> PRT
   <213> Artificial Sequence
<220>
   <223> Tag sequence
<400> 5
   Asp Asp Asp Asp Asp Cys Asp
   1               5
<210> 6
   <211> 5
   <212> PRT
   <213> Artificial Sequence
<220>
   <223> Tag sequence
<400> 6
   Lys Lys Cys Lys Lys
   1               5
<210> 7
   <211> 5
   <212> PRT
   <213> Artificial Sequence
<220>
   <223> Tag sequence
<400> 7
   Arg Arg Cys Arg Arg
   1               5
<210> 8
   <211> 5
   <212> PRT
   <213> Artificial Sequence
<220>
   <223> Tag sequence
<400> 8
   Asp Asp Cys Asp Asp
   1               5
<210> 9
   <211> 21
   <212> PRT
   <213> Artificial Sequence
<220>
   <223> Tag sequence
<400> 9
   Asp Cys Asp Ala Ala Ala Ala Ala Ala Ala Ala Ala Ala Ala Ala Ala
   1               5                   10                  15
   Ala Ala Ala Ala Ala
               20
<210> 10
   <211> 21
   <212> PRT
   <213> Artificial Sequence
<220>
   <223> Tag sequence
<400> 10
   Ala Ala Ala Ala Ala Ala Ala Ala Ala Ala Ala Ala Ala Ala Ala Ala
   1               5                   10                  15
   Ala Ala Asp Cys Asp
               20
<210> 11
   <211> 21
   <212> PRT
   <213> Artificial Sequence
<220>
   <223> Tag sequence
<400> 11
   Ala Ala Ala Ala Ala Ala Ala Ala Ala Asp Cys Asp Ala Ala Ala Ala
   1               5                   10                  15
   Ala Ala Ala Ala Ala
               20
<210> 12
   <211> 11
   <212> PRT
   <213> Artificial Sequence
<220>
   <223> Tag sequence
<400> 12
   Ala Ala Ala Ala Asp Cys Asp Ala Ala Ala Ala
   1               5                   10
<210> 13
   <211> 5
   <212> PRT
   <213> Artificial Sequence
<220>
   <223> Tag sequence
<400> 13
   Ala Asp Cys Asp Ala
   1               5
<210> 14
   <211> 7
   <212> PRT
   <213> Artificial Sequence
<220>
   <223> Tag sequence
<400> 14
   Val Pro Arg Asp Cys Gly Gly
   1               5
<210> 15
   <211> 6
   <212> PRT
   <213> Artificial Sequence
<220>
   <223> Tag sequence
<400> 15
   Cys Lys Pro Cys Ile Cys
   1               5
<210> 16
   <211> 6
   <212> PRT
   <213> Artificial Sequence
<220>
   <223> Tag sequence
<400> 16
   Cys Lys Pro Ser Ile Ser
   1               5

## Description

### TECHNICAL FIELD

The present disclosure includes a fusion polypeptide and a method for producing a fusion polypeptide.

### BACKGROUND

K. Stubenrauch, Bachmannb, Rudolpha, H. Lilie; Journal of Chromatography B, 737 (2000) 77-841 describes that, when recombinant VP1 protein is expressed in E. coli and purified by ion exchange chromatography according to a conventional protein production protocol, the yield of the recombinant VP1 protein is deteriorated. Then, Non-Patent Document 1 also describes that a sequence containing oligoglutamate sequences (E6, E8, E10) immediately after cysteine is inserted near a HI-loop of the VP1 protein to prevent aggregation of the recombinant VP1 protein to form virus-like particles. Several database UniProt entries disclose fusion polypeptides comprising a target polypeptide and a tag polypeptide, such as database accession nos. AOA2H3EBQ4, AOA084VVX5, A0A0K6GZJ0 and X1WJM7.

There is a problem that, when producing a polypeptide by gene recombination, a free thiol group contained in cysteine of the expressed polypeptide is unstable and binds to other thiol groups, resulting that it is easy to form a multimer of the produced polypeptide.

An object of the present invention is to maintain a thiol group in a polypeptide in a reduced state as much as possible when the polypeptide is expressed and recovered by gene recombination in a host cell.

### SUMMARY

An aspect of the present disclosure is a fusion polypeptide containing a target polypeptide and a tag polypeptide, in which the tag polypeptide is present on an N-terminal side and/or a C-terminal side of the target polypeptide and comprises a structure represented by Xaa-Cys-Zaa. In this fusion polypeptide, (1) Xaa is Asp or Glu, and Zaa is Asp or Glu, or (2) Xaa is Lys or Arg, and Zaa is Lys or Arg, and wherein the target polypeptide is an antibody or an antibody fragment,and wherein the antibody fragment is an Fab of the antibody, an Fab' of the antibody, a heavy chain of the antibody or a light chain of the antibody. The invention also relates to a fusion polypeptide wherein the tag polypeptide has a structure represented by (Paa)m-Xaa-Cys-Zaa-(Qaa)n, wherein Paa and Qaa each represent an amino acid, m and n each independently represent a number of amino acids selected from integers of 0 to 20, and (Paa)m-Xaa and Zaa-(Qaa)n have an identical electrical polarity. The invention also relates to a fusion polypeptide wherein the electrical polarity of (Paa)m-Xaa and Zaa-(Qaa)n is negative; or the electrical polarity of (Paa)m-Xaa and Zaa-(Qaa)n is positive. In another aspect of the invention, the tag polypeptide of the fusion polypeptide is bound to the C-terminal side of the target polypeptide. Also encompassed is a fusion polypeptide wherein the electrical polarity of (Paa)m-Xaa and Zaa-(Qaa)n is negative, m is selected from integers of 1 to 20, and the amino acid present immediately before Xaa is Asp or Glu. Alternatively the invention relates to fusion polypeptide wherein the electrical polarity of (Paa)m-Xaa and Zaa-(Qaa)n is negative, n is selected from integers of 1 to 20, and the amino acid present immediately after Zaa is Asp or Glu. Also encompassed is a fusion polypeptide wherein at least one selected from the group consisting of (Paa)m and (Qaa)n does not comprise a basic amino acid. Further encompassed is a fusion polypeptide wherein the electrical polarity of (Paa)m-Xaa and Zaa-(Qaa)n is positive, m is selected from integers of 1 to 20, and the amino acid present immediately before Xaa is Lys or Arg, or the amino aid present immediately after Zaa is Lys or Arg. The invention also relates to fusion polypeptides wherein at least one selected from the group consisting of (Paa)m and (Qaa)n does not comprise an acidic amino acid. Further encompassed is a fusion protein wherein a labeling substance is bound to Cys in the Xaa-Cys-Zaa. Still further encompassed is a fusion polypeptide wherein Xaa is Asp, and Zaa is Asp.

Accordingly, the invention also relates to methods for producing a fusion polypeptide, the method comprising steps of producing a fusion polypeptide using a DNA encoding the fusion polypeptide according to any one of claims 1 to 12, and recovering an expressed fusion polypeptide. Further encompassed is a method further comprising refolding the fusion protein recovered from the host cell.

### BRIEF DESCRIPTION OF THE DRAWINGS

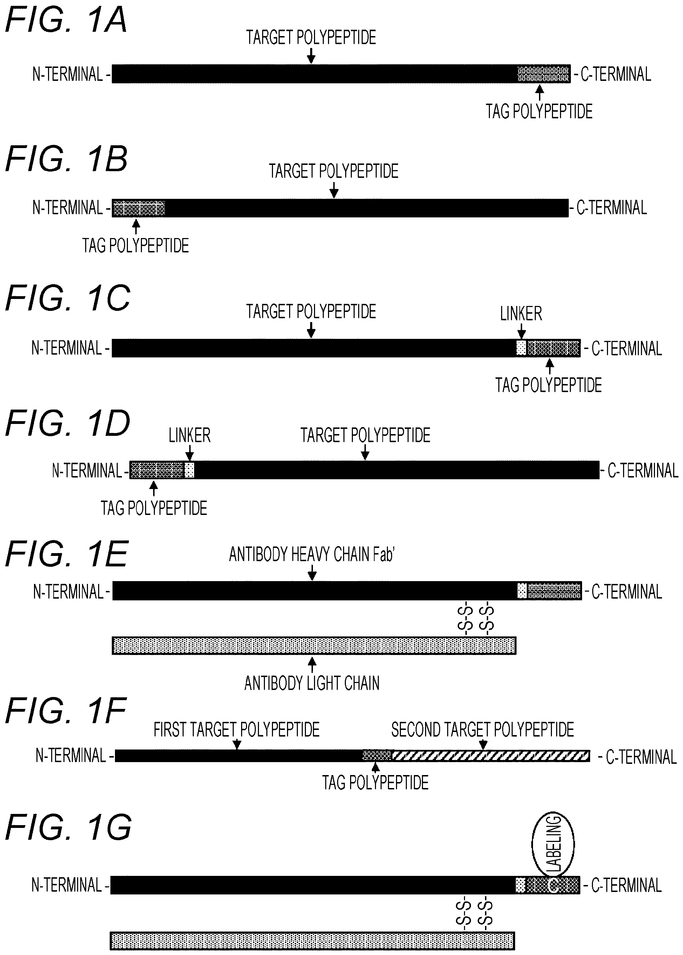

Fig. 1A shows a structural example of a fusion polypeptide containing a tag polypeptide on a C-terminal side of a target polypeptide. Fig. 1B shows a structural example of a fusion polypeptide containing a tag polypeptide on a N-terminal side of a target polypeptide. Fig. 1C shows a structural example of a fusion polypeptide containing a linker and a tag polypeptide on the C-terminal side of a target polypeptide. Fig. ID shows a structural example of a fusion polypeptide containing a linker and a tag polypeptide on the N-terminal side of a target polypeptide. Fig. IE shows an example of Fab' of an antibody containing Fab' of a heavy chain of the antibody and a light chain of the antibody, as an example of a fusion polypeptide containing two or more peptide units. Fig. 1F shows a structural example of a fusion polypeptide containing a first target polypeptide, a tag polypeptide, and a second target polypeptide from the N-terminal side of a fusion polypeptide. Fig. 1G shows an example in which a labeling substance is bound to cysteine of a tag polypeptide.

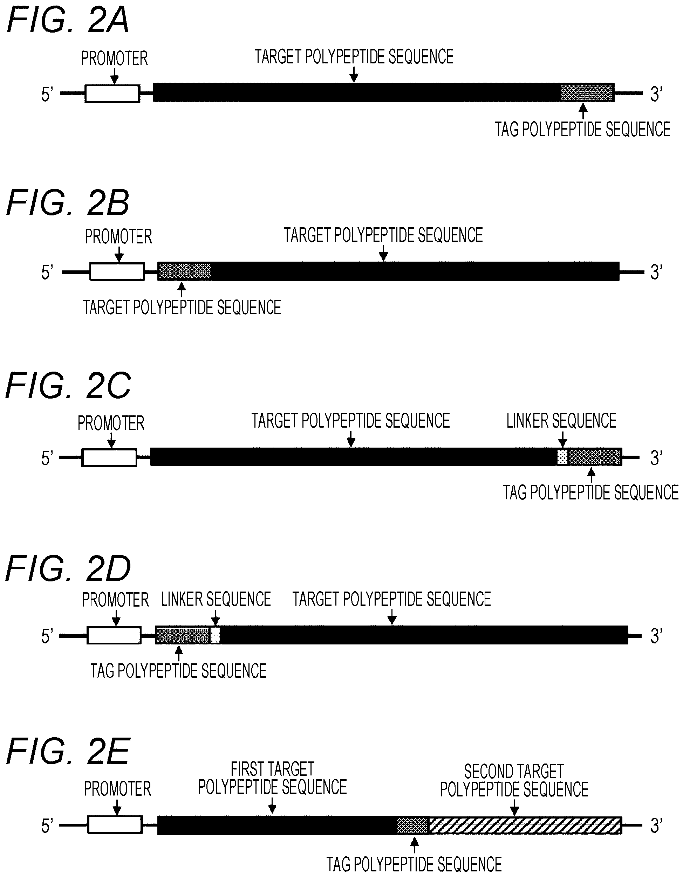

Fig. 2A shows a structural example of a nucleic acid encoding a fusion polypeptide containing a tag polypeptide on the C-terminal side of a target polypeptide. Fig. 2B shows a structural example of a nucleic acid encoding a fusion polypeptide containing a tag polypeptide on the N-terminal side of a target polypeptide. Fig. 2C shows a structural example of a nucleic acid encoding a fusion polypeptide containing a linker and a tag polypeptide on the C-terminal side of a target polypeptide. Fig. 2D shows a structural example of a nucleic acid encoding a fusion polypeptide containing a linker and a tag polypeptide on the N-terminal side of a target polypeptide. Fig. 2E shows a structural example of a nucleic acid encoding a fusion polypeptide containing a first target polypeptide, a tag polypeptide, and a second target polypeptide.
Fig. 3 shows an SDS-PAGE image of a fusion polypeptide expressed in E. coli.
Fig. 4 shows a western blotting image of a fusion polypeptide expressed in E. coli.
Fig. 5 shows a western blotting image of a fusion polypeptide expressed in E. coli.
Fig. 6 shows results of ELISA using Fab' expressed as a fusion polypeptide.
Fig. 7 shows an SDS-PAGE image of a fusion polypeptide expressed in E. coli.

### DETAILED DESCRIPTION OF THE PREFERRED EMBODIMENTS

### [Fusion Polypeptide]

A first embodiment of the present disclosure relates to a fusion polypeptide.

As shown in Figs. 1A and 1B, the fusion polypeptide contains at least a target polypeptide and a tag polypeptide.

### <Target polypeptide>

The target polypeptide intends a polypeptide corresponding to an intended use of the fusion polypeptide. The number of amino acid residues constituting the target polypeptide is not particularly limited. For example, it may be an oligopeptide of several residues, or a protein of hundreds to tens of thousands of residues.

The target polypeptide is an antibody or an antibody fragment selected from an Fab of the antibody, Fab' of the antibody, a heavy chain or light chain of the antibody. The heavy chain or light chain may be a part of a heavy chain and/or light chain containing at least a portion of a variable region. More preferably, examples of the target polypeptide can include Fab of the antibody, Fab' of the antibody, and a Fab portion or Fab' portion of the heavy chain. The Fab of the antibody intends a fragment in which the Fab portion of the heavy chain and the light chain are bound to each other. The Fab' of the antibody intends a fragment in which the Fab' portion of the heavy chain and the light chain are bound to each other. The Fab portion of the heavy chain intends a heavy chain fragment that is an antigen-binding region that does not contain two cysteines that serve as a hinge portion of the antibody and is not bound to the light chain. The Fab' portion of the heavy chain intends a heavy chain fragment that is an antigen-binding region that contains two cysteines that serve as a hinge portion of the antibody and is not bound to the light chain.

Examples of origins of the heavy chain or light chain can include human, mouse, rat, chicken, dog, cat, sheep, guinea pig, goat, pig, and the like. Further, the heavy chain or light chain may be obtained by modifying an amino acid sequence derived from an animal species other than human into a human amino acid sequence. The heavy chain or light chain may be a chimera having amino acid sequences of two or more heterogeneous animals.

### <Tag Polypeptide>

The tag polypeptide comprises a structure represented by Xaa-Cys (cysteine)-Zaa. Xaa and Zaa each represent one amino acid. "Zaa" is distinguished from "Z" representing "glutamic acid or glutamine" in the one-letter code of amino acid. Xaa intends the N-terminal side, and Zaa intends the C-terminal side. In one embodiment, both Xaa and Zaa are acidic amino acids. In this embodiment, Xaa can be Asp or Glu, and Zaa can be Asp or Glu. In another embodiment, both Xaa and Zaa are basic amino acids. In this embodiment, Xaa can be Lys or Arg, and Zaa can be Lys or Arg. In the present disclosure, an amino acid is sometimes represented by a one-letter code, in addition to a three-letter code. In one-letter code, cysteine is represented by "C", aspartic acid is represented by "D", glutamic acid is represented by "E", lysine is represented by "K", arginine is represented by "R", and alanine is represented by "A". In particular embodiments Xaa and Zaa are identical and are one selected from Asp, Glu, Lys or Arg.

Preferably, the tag polypeptide has a structure represented by (Paa)ₘ-Xaa-Cys-Zaa-(Qaa)ₙ. "Paa" represents one amino acid. "Paa" is distinguished from "P" representing "proline" in the one-letter code of amino acid. "Qaa" represents one amino acid. "Qaa" is distinguished from "Q" representing "glutamine" in the one-letter code of amino acid. X and Z each represent an amino acid, and m and n each independently represent the number of amino acids selected from an integer of 0 to 20, and preferably 1 to 15. More preferably, m and n each independently represent the number of amino acids selected from 1, 2, 3, 4, 5, 6, 7, 8, 9, 10, 11, 12, 13, 14, and 15. In particular embodiments, n+m is at least 3, most preferably n+m is at least 4.

(Paa)ₘ-Xaa and Zaa-(Qaa)n preferably have the same electrical polarity. (Paa)m-Xaa and Zaa-(Qaa)ₙ have the same electrical polarity, whereby they are expected to repel each other and prevent a thiol group of the sandwiched cysteine from binding to other groups. Therefore, the electrical polarity of (Paa)ₘ-Xaa and Zaa-(Qaa)ₙ may be negative or positive.

Based on a pKa value of a side chain of each amino acid contained in (Paa)ₘ-Xaa or Zaa-(Qaa)ₙ, the electrical polarity can be obtained by calculating an effective charge of (Paa)ₘ-Xaa or Zaa-(Qaa)ₙ at a pH of a solution in which the tag polypeptide is present. When the effective charge is a positive value, the electrical polarity is positive, and when the effective charge is a negative value, the electrical polarity is negative. The effective charge of (Paa)ₘ-Xaa and the effective charge of Zaa-(Qaa)ₙ need not be equal.

When the electrical polarity of (Paa)ₘ-Xaa and Zaa-(Qaa)ₙ is negative, Xaa is Asp or Glu, m is 1 or more, and the amino acid present immediately before Xaa, that is, the amino acid adjacent to the N-terminal side of Xaa is preferably Asp or Glu. Moreover, when the electrical polarity of (Paa)ₘ-Xaa and Zaa-(Qaa)ₙ is negative, Zaa is Asp or Glu, n is 1 or more, and the amino acid present immediately after Zaa, that is, the amino acid adjacent to the C-terminal side of Zaa is preferably Asp or Glu. Further, when the electrical polarity of (Paa)ₘ-Xaa and Zaa-(Qaa)ₙ is negative, it is more preferable that (Paa)ₘ and/or (Qaa)ₙ does not contain a basic amino acid.

When the electrical polarity of (Paa)ₘ-Xaa and Zaa-(Qaa)ₙ is positive, Xaa is Lys or Arg, m is 1 or more, and the amino acid present immediately before Xaa, that is, the amino acid adjacent to the N-terminal side of Xaa is preferably Lys or Arg. Moreover, when the electrical polarity of (Paa)ₘ-Xaa and Zaa-(Qaa)ₙ is positive, Zaa is Lys or Arg, n is 1 or more, and the amino acid present immediately after Zaa, that is, the amino acid adjacent to the C-terminal side of Zaa is preferably Lys or Arg. Further, when the electrical polarity of (Paa)ₘ-Xaa and Zaa-(Qaa)ₙ is positive, it is more preferable that (Paa)ₘ and/or (Qaa)ₙ does not contain an acidic amino acid. In particular embodiments all of Paa, Xaa, Zaa and Xaa are identical and are one selected from Asp, Glu, Lys or Arg.

The polarity of amino acids is as follows. Acidic amino acids and basic amino acids are sometimes collectively referred to as polar amino acids.
Acidic amino acids: Asp (aspartic acid), Glu (glutamic acid)
Basic amino acids: Arg (arginine), Trp (tryptophan),
His (histidine), Lys (lysine)
(Paa)ₘ and/or (Qaa)ₙ preferably does not contain Cys.

### <Structure of Fusion Polypeptide>

In the fusion polypeptide, the tag polypeptide is present on the N-terminal side or the C-terminal side of the target polypeptide. Fig. 1A shows an example in which the tag polypeptide is present on the N-terminal side of the target polypeptide. Fig. 1B shows an example in which the tag polypeptide is present on the C-terminal side of the target polypeptide. Preferably, the tag polypeptide is present in the C-terminal region of the target polypeptide.

The tag polypeptide and the target polypeptide may be directly bonded. The tag polypeptide and the target polypeptide may be bonded via a linker as shown in Figs. 1C and ID. The linker may be any sequence not contained in the target polypeptide and tag polypeptide. For example, the linker may have a second tag sequence such as a polyhistidine tag sequence, a c-myc tag sequence, a FLAG (registered trademark) tag sequence, an HA tag sequence, a V5 sequence, a thioredoxin sequence, a glutathione-S-transferase sequence, or a Solubility Enhancement Tags (SET) sequence. The linker can contain at least one amino acid. The linker preferably contains 1 to 20 amino acids, and more preferably contains 1 to 15 amino acids.

The fusion polypeptide may consist of a single peptide unit as shown in Figs. 1A to 1D. The fusion polypeptide may also consist of two or more peptide units as shown in Fig. IE. Fig. IE shows an example of Fab' of the antibody. The number of peptide units contained in the fusion polypeptide is not limited as long as the function of the target polypeptide contained in the fusion polypeptide can be exhibited. When the fusion polypeptide contains two or more peptide units, the tag polypeptide may be contained in any one unit, or the tag polypeptide may also be contained in a plurality of units.

The fusion polypeptide may contain sequences other than the target polypeptide and tag polypeptide on the N-terminal side or the C-terminal side.

In one embodiment, the fusion polypeptide contains a first target polypeptide, a tag polypeptide, and a second target polypeptide. That is, in this fusion polypeptide, the tag polypeptide is sandwiched between two types of target polypeptides (Fig. IF). The first target polypeptide and the second target polypeptide are polypeptides derived from polypeptides constituting a specific type of protein. The first target polypeptide and the second target polypeptide can be polypeptides having the same amino acid sequence or a different amino acid sequence. Alternatively, the first target polypeptide and the second target polypeptide can be polypeptides derived from different types of proteins. For example, the first target polypeptide can be a polypeptide derived from a desired protein, and the second target polypeptide can be a polypeptide that improves solubility of the fusion polypeptide. Further, the amino acid sequence of the tag polypeptide may be inserted in the middle of the amino acid sequence of the polypeptide constituting one unit contained in a specific protein. Specifically, the amino acid sequence of the tag polypeptide can be inserted into a loop region (HI loop, DE loop, or the like) of VP1 protein of SV40. In this case, the fusion polypeptide contains a polypeptide on the N-terminal side of VP1 as the first target polypeptide, a tag polypeptide, and a polypeptide on the C-terminal side of VP1 as the second target polypeptide.

The fusion polypeptide may contain sugar chain modification.

Cysteine present within a structure represented by Xaa-Cys-Zaa in the tag polypeptide contained in the fusion polypeptide may be labeled with a labeling substance as shown in Fig. 1G. The term "label" means that a labeling substance is bound directly or indirectly by chemical bonding. Examples of the labeling substance include haptens, particles, signal generating substances, enzymes, fluorescent substances, and the like. Examples of haptens include biotin, dinitrophenol (DNP), and the like. Examples of the particles include metal particles, polystyrene particles, latex particles, and the like. The particles may have magnetism. Examples of the signal generating substance include fluorescent substances, luminescent substances, radioactive isotopes, and the like. Examples of the fluorescent substance include fluorescent dyes such as fluorescein isothiocyanate (FITC), rhodamine and Alexa Fluor (registered trademark), fluorescent proteins such as GFP, and the like. Enzymes are substances that catalyze chemical reactions of other substances to generate signals, and specific examples include alkaline phosphatase, peroxidase, β-galactosidase, luciferase, and the like.

The labeling substance can be bound to the fusion polypeptide via a thiol group of cysteine. A method for binding a labeling substance to a thiol group is publicly known as follows.

The thiol group of cysteine can be bound to a labeling substance by cross-linking with a compound having a maleimide group or a bromo (or iodo) acetamide group as a reactive group (see the figure below). For example, when a thiol group of cysteine of a tag polypeptide and a labeling substance having a thiol group are cross-linked, a cross-linking reagent or linker having maleimide at both ends may be used.

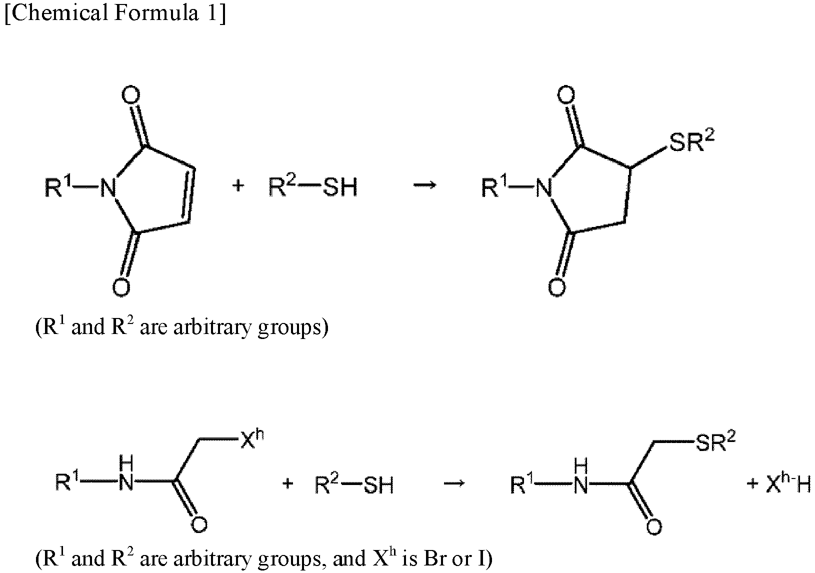

The above cross-linking reaction can be performed under normal temperature and normal pressure. The solvent used in the reaction is not particularly limited as long as it is inert to the above reaction and each compound to be subjected to the reaction can be lysed or dispersed. Examples of the solvent include aromatic hydrocarbons such as benzene, toluene and xylene, ethers such as diethyl ether, tetrahydrofuran, ethylene glycol dimethyl ether and 1,4-dioxane, and amides such as N,N-dimethylformamide, sulfoxides such as dimethyl sulfoxide, halogenated hydrocarbons such as dichloromethane and chloroform, and the like. These solvents can be used alone or as a mixture.

### [Method for Producing Fusion Polypeptide]

The second aspect of the present disclosure relates to a method for producing a fusion polypeptide described in the first embodiment. In particular embodiments, the method is a method for producing a fusion polypeptide comprising a protected thiol group, the method comprising (a) generating a fusion polypeptide between the target polypeptide and a tag polypeptide comprising a structure represented by Xaa-Cys-Zaa, wherein (1) Xaa is Asp or Glu, and Zaa is Asp or Glu, or (2) Xaa is Lys or Arg, and Zaa is Lys or Arg, wherein the tag polypeptide is present on an N-terminal side and/or a C-terminal side of the target polypeptide, and (b) recombinantly expressing said fusion polypeptide. The method for producing a fusion polypeptide is not limited as long as the desired fusion polypeptide is obtained. For example, production of a fusion polypeptide can be performed using genetic engineering techniques. The production of a fusion polypeptide can be performed with a cell-free system or cells using a DNA encoding the fusion polypeptide. The method for producing a fusion polypeptide at least includes steps of producing a fusion polypeptide using a DNA encoding the fusion polypeptide, and recovering the expressed fusion polypeptide.

### <Preparation of Expression Vector>

Described but not claimed herein is a nucleic acid (DNA) encoding an amino acid sequence of the fusion polypeptide is prepared. The nucleic acid encoding the fusion polypeptide inserts, for example, a DNA encoding a complementary strand sequence of a cDNA sequence for expressing a target polypeptide (hereinafter simply referred to as "encoding a target polypeptide") and a DNA encoding a tag sequence into an expression vector to construct an expression vector that expresses the fusion polypeptide. When the tag polypeptide is present on the C-terminal side of the fusion polypeptide, as shown in Fig. 2A, in the vector, a sequence encoding a target polypeptide and a sequence encoding a tag polypeptide are arranged from the 5' side at the downstream of the promoter. When the tag polypeptide is present on the N-terminal side of the fusion polypeptide, as shown in Fig. 2B, in the vector, a sequence encoding a tag polypeptide and a sequence encoding a target polypeptide are arranged from the 5' side at the downstream of the promoter. In a case where a linker is present between the target polypeptide and the tag polypeptide, when the tag polypeptide is present on the C-terminal side of the fusion polypeptide, as shown in Fig. 2C, in the vector, a sequence encoding a target polypeptide, a sequence encoding a linker, and a sequence encoding a tag polypeptide are arranged from the 5' side at the downstream of the promoter. In a case where a linker is present between the target polypeptide and the tag polypeptide, when the tag polypeptide is present on the N-terminal side of the fusion polypeptide, as shown in Fig. 2D, in the vector, a sequence encoding a tag polypeptide, a sequence encoding a linker, and a sequence encoding a target polypeptide are arranged from the 5' side at the downstream of the promoter. When the fusion polypeptide contains a first target polypeptide, a tag polypeptide, and a second target polypeptide, as shown in Fig. 2E, in the vector, a sequence encoding a first target polypeptide, a sequence encoding a tag polypeptide, and a sequence encoding a second target polypeptide, are arranged from the 5' side at the downstream of the promoter.

The sequence encoding a target polypeptide and the sequence encoding a tag polypeptide may be inserted into an expression vector separately. The sequence encoding a target polypeptide and the sequence encoding a tag polypeptide may be inserted as a nucleic acid in which the sequence encoding a target polypeptide and the sequence encoding a tag polypeptide are connected.

The sequence encoding a linker may be a sequence included in the expression vector in advance, or the sequence encoding a linker may be added to the sequence encoding a tag polypeptide or the sequence encoding a target polypeptide. Alternatively, it may be inserted as a nucleic acid in which the sequence encoding a target polypeptide, the sequence encoding a linker, and the sequence encoding a tag polypeptide are connected.

As the expression vector, a known one can be selected. Specific examples of the vector can include plasmid vectors, cosmid vectors, phage vectors, viral vectors (for example, adenoviral vectors, adeno-associated viral vectors, retroviral vectors (including lentiviral vectors), herpes viral vectors, and the like) and the like.

Examples of cell-free expression systems and vectors when a host is E. coli include plasmid vectors such as pBlueScript, pBR322 or variants thereof (for example, pB325, pAT153, pUC8, and the like), pUC19, pGEM-T, pCR-Blunt, pTA2, and pET, M13 phage or variants thereof, λ phage or variants thereof, and the like. Preferably, the vector contains an auxotrophic marker, a drug resistance marker, an expression promoter sequence other than a promoter that expresses a fusion polypeptide, and an expression terminator sequence. More preferably, the vector contains an ampicillin resistance gene derived from pBlueScript, and a lactose promoter. It is also possible to use a vector suitable for self-cloning.

When yeast is used as a host, plasmid vectors such as pYepSecl, pMFa, and pYES2 can be used. When insect cells are used as hosts, for example, plasmid vectors such as pAc and pVL can be used. When mammalian cells are used as hosts, for example, plasmid vectors such as pCDM8 and pMT2PC can be used, but are not limited thereto.

The method for constructing an expression vector for expressing the fusion polypeptide described above is publicly known.

### <Cell-Free Expression System>

When the fusion polypeptide is expressed using a cell-free expression system, first, an RNA encoding a fusion polypeptide is obtained. The RNA can be obtained by cleaving a vector containing a DNA encoding the fusion polypeptide with a restriction enzyme downstream from the 3' end of the fusion polypeptide sequence to linearize the vector, and performing a transcription reaction using a transcriptase that can translate from a promoter present on the 5' end side of the fusion polypeptide. In this case, T7 promoter, T3 promoter, SP6 promoter or the like can be used as a promoter. Transcriptases corresponding to the above promoters are T7 RNA polymerase, T3 RNA polymerase, and SP6 RNA polymerase. RNA obtained by transcription reaction is mixed with cell-free extracts (containing ribosome, translation factors, tRNA, and the like) that maintain a translation function extracted from E. coli, rabbit reticulocyte lysate (RRL), wheat germ extract, insect cells (such as SF9 or SF21), and the like, an energy source (ATP) and an amino acid, and the mixture is incubated at a predetermined temperature for a predetermined time to produce a fusion polypeptide.

### <Expression System Using Host Cells>

Examples of host cells into which an expression vector for expressing a fusion polypeptide is introduced can include E. coli, yeast, insect cells, and mammalian cells. The method for introducing an expression vector into these host cells and a method for culturing host cells are publicly known.

A method for inducing expression of a fusion polypeptide inserted into an expression vector in the host cells into which the expression vector has been introduced is also publicly known. For example, when the host has a DE3 sequence in a genome, it has a Lac operator sequence as a promoter for expressing T7 RNA polymerase or T3 RNA polymerase, so that expression of T7 RNA polymerase or T3 RNA polymerase can be induced by isopropyl-β-thiogalactopyranoside. In such host cells, the expression of the fusion polypeptide can be controlled by the presence or absence of addition of isopropyl-β-thiogalactopyranoside.

### <Recovery and Folding of Expressed Fusion Polypeptide>

The expressed fusion polypeptide can be recovered according to a known method. The recovered fusion polypeptide can be refolded if necessary. Refolding methods are also publicly known.

When the expressed fusion polypeptide is present in extracellular fractions such as the medium, the extracellular fractions and the host cells are separated by centrifugation or the like, and then the extracellular fractions containing the fusion polypeptide are recovered as a fusion polypeptide roughly purified solution.

When E. coli is used as the host cell, the expressed fusion polypeptide is sometimes present in a periplasmic fraction. In this case, for example, the recovered host cells are suspended in a periplasmic extraction buffer such as sucrose buffer including sucrose, Tris-HCl buffer, EDTA and the like, and an osmotic shock with magnesium sulfate is applied, whereby the periplasmic fraction can be extracted. The extract of the periplasmic fraction can be recovered as a roughly purified solution of the fusion polypeptide.

When the expressed fusion polypeptide is present in soluble fractions of the host cells, the host cells are suspended in a cell lysis buffer containing a surfactant or the like, and the suspension is crushed with ultrasound or the like, if necessary, and a supernatant obtained by centrifugation or the like can be recovered as a roughly purified solution of the fusion polypeptide.

When the expressed fusion polypeptide is present in insoluble fractions (solid fraction or inclusion body after lysing total cells with a cell lysis buffer or the like, and the like) of the host cells, the insoluble fractions are suspended and lysed in a denaturing buffer such as urea or guanidine hydrochloride or the like, then the lysate is dialyzed to remove a denaturant component, whereby a roughly purified solution of the fusion polypeptide can be obtained.

Purification of the fusion polypeptide from the recovered roughly purified solution of the fusion polypeptide can be performed by gel filtration, ion exchange chromatography or the like. When the fusion polypeptide has a linker and the above-described second tag sequence is present in the linker, the fusion polypeptide can be purified by performing affinity purification according to the second tag sequence.

Further, when the purified fusion polypeptide is not correctly folded, refolding can be performed. Refolding can be performed by, for example, lysing the purified fusion polypeptide in a denaturing buffer including urea, guanidine hydrochloride or the like or adding a denaturant such as urea or guanidine hydrochloride to the roughly purified solution of the fusion polypeptide to lyse aggregation of the fusion polypeptide, and then performing, for example, dilution refolding, dialysis refolding, solid phase refolding, size exclusion chromatography refolding, surfactant refolding, or the like, described in Arakawa and Ejima (Antibodies 2014, 3, 232-241; doi: 10.3390/antib3020232). Dialysis refolding is preferable.

For example, in order to produce a Fab portion and Fab' portion of the antibody, a fusion polypeptide having a Fab portion (or Fab' portion) of a heavy chain of the antibody as a target polypeptide and a light chain of the antibody are each expressed in E. coli in different flasks, E. coli is recovered from each flask, and the periplasmic fraction of each E. coli is lysed in a denaturing buffer (preferably, a 6 M guanidine hydrochloride buffer). The obtained denaturing buffer lysates are mixed so that the expressed fusion polypeptide and the light chain of the antibody are almost the same by molar ratio, and the mixed lysate is dialyzed according to the dialysis refolding described in, for example, Arakawa and Ejima, whereby the Fab portion (or Fab' portion) of an antibody in which the Fab portion (or Fab' portion) of the heavy chain and the light chain bound to each other contained in the fusion polypeptide can be recovered.

The dialysis conditions can be appropriately changed according to a purification scale and the type of the target polypeptide. Dialysis for refolding the Fab portion (or Fab' portion) of the antibody can be performed under conditions of dialysate 1 [3 M to 3.5 M guanidine hydrochloride; about 50 mM Tris-HCl buffer (pH 7.8 to 8.2); about 1 mM EDTA] for about 6 to 10 hours, dialysate 2 [2 M to 2.5 M guanidine hydrochloride; about 50 mM Tris-HCl buffer (pH 7.8 to 8.2); about 1 mM EDTA] for about 10 to 14 hours, dialysate 3 [1 M to 1.5 M guanidine hydrochloride; 0.3 M to 0.5 M arginine hydrochloride; about 50 mM Tris-HCl buffer (pH 7.8 to 8.2); about 1 mM EDTA; disulfide exchange reagent (1 mM GSH/1 mM GSSG)] for about 10 to 14 hours, dialysate 4 [0.3 M to 0.75 M guanidine hydrochloride; 0.3 M to 0.5 M arginine hydrochloride; about 50 mM Tris-HCl buffer (pH 7.8 to 8.2); about 1 mM EDTA; disulfide exchange reagent (1 mM GSH/1 mM GSSG)] for about 10 to 14 hours, and dialysate 5 [about 50 mM Tris-HCl buffer (pH 7.8 to 8.2); about 1 mM EDTA] for about 10 to 14 hours.

Furthermore, the method for producing a fusion polypeptide may include a step of binding Cys in the Xaa-Cys-Zaa contained in the tag polypeptide and a labeling substance. Regarding the "labeling substance" and the binding of the labeling substance, description in the section of [Fusion Polypeptide] is incorporated herein.

### EXAMPLES

Hereinafter, embodiments of the present disclosure will be described in more detail by way of examples, but the embodiments of the present disclosure are not to be construed as being limited to the examples.

### 1. Example 1

A fusion polypeptide in which tag polypeptides of SEQ ID NOs: 1 to 5 shown in Table 1 were connected to the C-terminal side of a target polypeptide maltose binding protein (MBP) via 6 histidine polyhistidine tags was expressed using pMAL-p5x E. coli expression system. In this system, MBP does not contain cysteine, so that aggregation properties of the fusion polypeptide will depend on cysteine in the tag polypeptides.

**[Table 1]**

| Tag polypeptide | SEQ ID NO: |
|---|---|
| DDDCDDD (D3CD3) | 1 |
| DDDDDDCDD (D6CD2) | 2 |
| DDDDCDDDD (D4CD4) | 3 |
| DDDDCDD (D4CD2) | 4 |
| DDDDDCD (D5CD) | 5 |

A periplasmic fraction containing the expressed fusion polypeptide was extracted by osmotic shock under ice-cooling using a sucrose buffer (30 mM Tris HCl, 20% sucrose, pH = 8, 1 mM EDTA) and 5 mM magnesium sulfate. The fusion polypeptide was purified from the extracted periplasmic fraction using amylose resin and an elution buffer (20 mM Tris-Cl, 200 mM NaCl, 1 mM EDTA, pH 7.4). The purified fusion polypeptide was analyzed by SDS-PAGE without reduction. As a control that does not form a disulfide bond, MBP combined with 15 amino acid peptide tag (Sib) designed by SibTech was used. As a control for forming a disulfide bond, Mouse Hinge (VPRDCGG: SEQ ID NO: 14) Heavy chain inter-chain disulphide sequence (M. Hinge) was used.

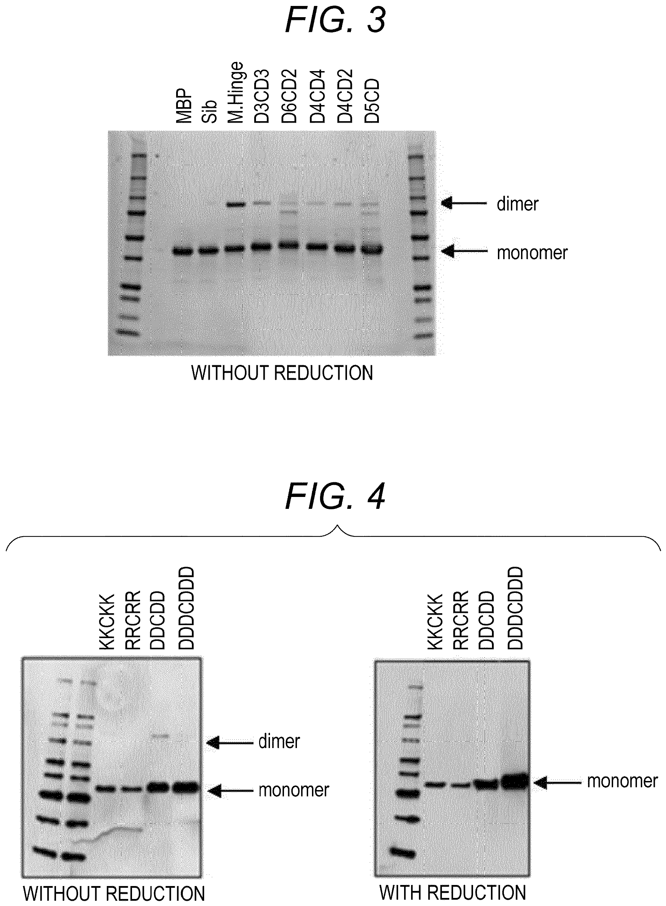

The results are shown in Fig. 3. Since MBP and Sib do not form a disulfide bond even in the non-reduced state, only a monomer band was detected. A dimer band was detected in M. Hinge. It is considered because M. Hinge contained cysteine in the sequence and formed a disulfide bond in the non-reduced state. In the fusion protein in which the tag polypeptides of SEQ ID NOs: 1 to 5 were bound, dimer formation was suppressed as compared with M. Hinge.

As to the results shown in Fig. 3, the monomer and dimer band densities of each polypeptide were quantified using Image J (National Institutes of Health, USA) to determine the monomer ratio. Furthermore, the relative ratio of the determined monomer ratio (Monomer ratio/M. Hinge) was calculated based on M. Hinge. The results are shown in Table 2.

**[Table 2]**

| | Monomer ratio/M. Hinge |
|---|---|
| MBP | 1.658 |
| Sib | 1.632 |
| M.Hinge | 1 |
| D3CD3 | 1.455 |
| D6CD2 | 1.586 |
| D4CD4 | 1.563 |
| D4CD2 | 1.528 |
| D5CD | 1.551 |

It was shown that, as compared with M. Hinge, the D3CD3, D6CD2, D4CD4, D4CD2 or D5CD tag polypeptide had an improved Monomer ratio/M. Hinge, indicating that aggregation was suppressed.

### 2. Example 2

Next, when MBP was used as the target polypeptide as in Example 1 and amino acids other than cysteine contained in the tag polypeptide were changed to basic amino acids, the number and position of aspartic acids was changed, and the monomer ratio was investigated by Western blotting. The investigation was performed by Western blotting. Anti-6-His Rabbit Polyclonal antibody was used as a primary antibody for Western blotting. Polyclonal Goat Anti Rabbit Immunoglobulin/HRP conjugated was used as a secondary antibody.

The sequence of the tag polypeptide used in Example 2 is shown in Table 3.

**[Table 3]**

| Tag polypeptide | SEQ ID NO: |
|---|---|
| KKCKK | 6 |
| RRCRR | 7 |
| DDCDD | 8 |
| DDDCDDD | 1 |

Fig. 4 shows the results of Western blotting.

For the results shown in Fig. 4, the monomer and dimer band densities of each polypeptide were quantified to determine the monomer ratio. The results are shown in Table 4.

**[Table 4]**

| | Monomer ratio (%) |
|---|---|
| KKCKK | 100 |
| RRCRR | 100 |
| DDCDD | 92.53082077 |
| DDDCDDD | 100 |

As shown in the left figure of Fig. 4, even the tag polypeptide of SEQ ID NO: 6 or SEQ ID NO: 7 in which the acidic amino acid aspartic acid was changed to the basic amino acid lysine or arginine could maintain the monomer state. In addition, as shown in SEQ ID NO: 8, the monomer ratio exceeded 92% even when the number of aspartic acids was reduced. From these results, it was suggested that polar amino acids are preferably present on both sides of cysteine.

In terms of the yield of the fusion polypeptide, comparing SEQ ID NOs: 6, 7, and 8 where two polar amino acids are present on both sides of cysteine, the fusion polypeptide containing the tag polypeptide of SEQ ID NO: 8 containing acidic amino acids showed a band more strongly in Fig. 4.

### 3. Example 3

Next, a Fab' portion of a HBs85 monoclonal antibody was constructed in vitro. As a target polypeptide, a fusion polypeptide in which glycine was sandwiched as a linker and the tag polypeptides shown in SEQ ID NOs: 1, 2, and 5 were connected to the C-terminal side of the Fab' portion of a heavy chain of the HBs85 monoclonal antibody was expressed in DE3-positive E. coli using a pET system. In addition, a light chain of the HBs85 monoclonal antibody was expressed in another E. coli. A periplasmic fraction of E. coli expressing the fusion polypeptide and a periplasmic fraction of E. coli expressing the light chain were extracted and each lysed in a denaturing buffer containing 6 M guanidine hydrochloride to prepare lysates. The obtained lysates were mixed so that the fusion polypeptide and the light chain were almost the same by molar ratio, and the mixed lysate was subjected to dialysis under the following conditions to bind the fusion polypeptide and the light chain while refolding. Dialysate 1 [3 M guanidine hydrochloride; 50 mM Tris-HCl buffer (pH 8.0); 1 mM EDTA] for 8 hours, dialysate 2 [2 M guanidine hydrochloride; 50 mM Tris-HCl buffer (pH 8.0); 1 mM EDTA] For 12 hours, dialysate 3 [1 M guanidine hydrochloride; 0.4 M arginine hydrochloride; 50 mM Tris-HCl buffer (pH 8.0); 1 mM EDTA; disulfide exchange reagent (1 mM GSH/1 mM GSSG)] for 12 hours, dialysate 4 [0.3 M guanidine hydrochloride; 0.4 M arginine hydrochloride; 50 mM Tris-HCl buffer (pH 8.0); 1 mM EDTA; disulfide exchange reagent (1 mM GSH/1 mM GSSG)] for 12 hours, and dialysate 5 [50 mM Tris-HCl buffer (pH 8.0); 1 mM EDTA] for 12 hours.

Dialysis was performed at 4°C.

The Fab' portion of the refolded HBs85 monoclonal antibody was evaluated by Western blotting under non-reducing conditions and reducing conditions. As an antibody, an HRP-labeled anti-mouse immunoglobulin antibody was used. As a control for forming a disulfide bond, original Mouse Hinge (M. Hinge WT: CKPCIC: SEQ ID NO: 15) was used. As a negative control that does not form a disulfide bond, HBs85 WT (that contains hinge sequence and tag polypeptide and does not form a dimer) was used.

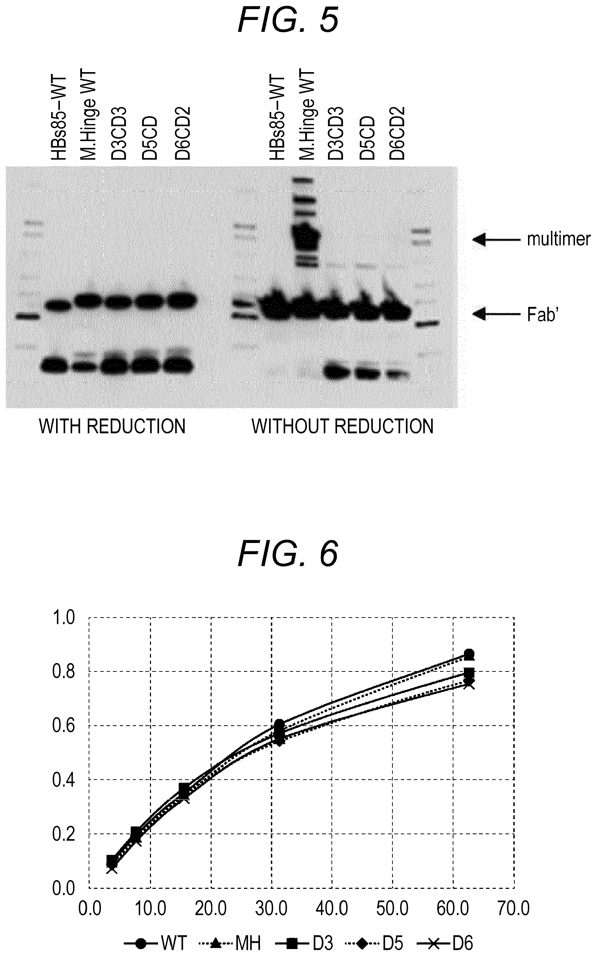

As shown in Fig. 5, in the non-reduced state, while M. Hinge WT showed strong aggregation, a complex of the fusion polypeptide containing the tag polypeptides shown in SEQ ID NOs: 1, 2, and 5 and the light chain was present without aggregation. For the results shown in Fig. 5, when the monomer and dimer band densities of each polypeptide were quantified to determine the monomer ratio, the monomer ratio of the Fab' portion containing the tag polypeptide shown in HBs85 WT and SEQ ID NO: 1, 2, or 5 was 100%.

Next, using the Fab' portion of the refolded HBs85 monoclonal antibody, binding to the antigen under non-reducing conditions was confirmed by ELISA.

Biotin was bound to the Fab' portion of the refolded HBs85 monoclonal antibody using a maleimide reaction, and a HBs antigen was detected by ELISA. The reactivity of each Fab' portion in ELISA is shown in Fig. 6. The reactivity was expressed as a relative ratio using M. Hinge WT as a negative control. The Fab' portion constructed using the fusion polypeptide showed the same reactivity as the Fab' portion constructed using HBs85-WT.

### 4. Example 4

Next, using MBP as the target polypeptide as in Example 1, whether there was an effect of maintaining the monomer ratio of the fusion polypeptide with respect to sequences of other tag polypeptides was investigated. In addition to the tag polypeptides represented by SEQ ID NOs: 1, 2, and 5, the sequences shown in Table 5 were investigated. In SEQ ID NOs: 9 to 13, a neutral amino acid alanine was inserted into the tag polypeptides. MBP was used as a control not forming a disulfide bond, and M. Hinge2 (CKPSIS: SEQ ID NO: 16) was used as a control forming a disulfide bond.

**[Table 5]**

| Tag polypeptide | SEQ ID NO: |
|---|---|
| DDDCDDD (D3CD3) | 1 |
| DDDDDCD (D5CD) | 5 |
| DDDDDDCDD (D6CD2) | 2 |
| DCDAAAAAAAAAAAAAAAAAA(DCDA18) | 9 |
| AAAAAAAAAAAAAAAAAADCD (A18DCD) | 10 |
| AAAAAAAAADCDAAAAAAAAA (A9DCDA9) | 11 |
| AAAADCDAAAA (A4DCDA4) | 12 |
| ADCDA | 13 |
| DCD | |
| ECE | |
| RCR | |
| KCK | |

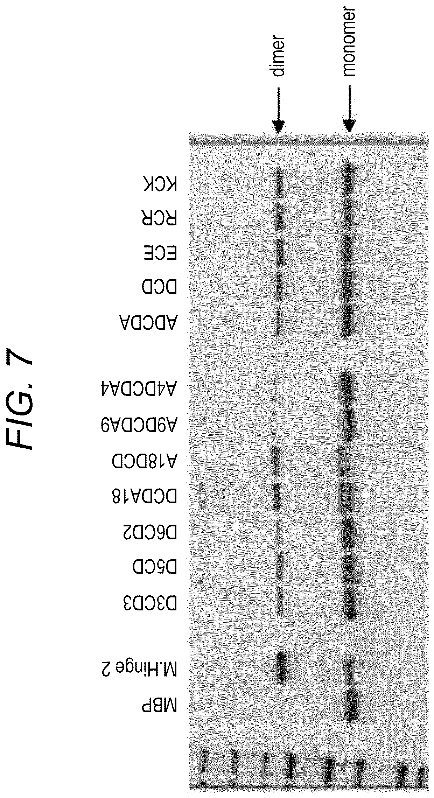

Fig. 7 shows the results of electrophoresis of the fusion polypeptides by SDS-PAGE. Also, for the results shown in Fig. 7, the monomer and dimer band densities of each polypeptide were quantified to determine the monomer ratio. Furthermore, the relative ratio of the determined monomer ratio (Monomer ratio/M. Hinge 2) was calculated based on M. Hinge 2. The results are shown in Table 6.

**[Table 6]**

| | Monomer ratio/M. Hinge 2 |
|---|---|
| MBP | 2.486 |
| M.Hinge 2 | 1 |
| D3CD3 | 2.002 |
| D5CD | 1.977 |
| D6CD2 | 2.134 |
| DCDA18 | 1.680 |
| A18DCD | 1.575 |
| A9DCDA9 | 2.290 |
| A4DCDA4 | 2.288 |
| ADCDA | 1.906 |
| DCD | 1.663 |
| ECE | 1.473 |
| RCR | 1.447 |
| KCK | 1.603 |

In Fig. 7, the investigated tag polypeptides all had a stronger monomer band than a dimer band, as compared with M. Hinge 2. Also in Table 6, the fusion polypeptides containing the investigated tag polypeptides showed a higher monomer ratio than the monomer ratio of M. Hinge 2. The higher the number of polar amino acids, the higher the monomer ratio tended to be. Even when a non-polar amino acid was inserted into the tag polypeptide, the monomer ratio was a higher value than that of M. Hinge 2, but in the tag polypeptides of SEQ ID NOs: 9 and 10, two monomer bands were detected in Fig. 7. This indicates that the A18 portion may be cleaved during recovery of the fusion polypeptide. On the other hand, the tag polypeptide of SEQ ID NO: 12 contained a total of 18 non-polar amino acids, 9 non-polar amino acids each sandwiching cysteine, but no cleavage was observed.

## Claims

1. A fusion polypeptide comprising a target polypeptide and a tag polypeptide, wherein
the tag polypeptide is present on a N-terminal side and/or a C-terminal side of the target polypeptide, and the tag polypeptide comprises a structure represented by Xaa-Cys-Zaa,
i) Xaa being Asp or Glu, and Zaa being Asp or Glu; or
ii) Xaa being Lys or Arg, and Zaa being Lys or Arg, and
wherein the target polypeptide is an antibody or an antibody fragment,and
wherein the antibody fragment is an Fab of the antibody, an Fab' of the antibody, a heavy chain of the antibody or a light chain of the antibody.

2. The fusion polypeptide according to claim 1, wherein the tag polypeptide has a structure represented by (Paa)ₘ-Xaa-Cys-Zaa-(Qaa)ₙ, wherein
Paa and Qaa each represent an amino acid,
m and n each independently represent a number of amino acids selected from integers of 0 to 20, and
(Paa)ₘ-Xaa and Zaa-(Qaa)ₙ have an identical electrical polarity.

3. The fusion polypeptide according to claim 2, wherein
the electrical polarity of (Paa)ₘ-Xaa and Zaa-(Qaa)ₙ is negative; or
the electrical polarity of (Paa)ₘ-Xaa and Zaa-(Qaa)ₙ is positive.

4. The fusion polypeptide according to any one of claims 1 to 3, wherein the tag polypeptide is bound to the C-terminal side of the target polypeptide.

5. The fusion polypeptide according to claim 3, wherein
the electrical polarity of (Paa)ₘ-Xaa and Zaa-(Qaa)ₙ is negative,
m is selected from integers of 1 to 20, and
the amino acid present immediately before Xaa is Asp or Glu.

6. The fusion polypeptide according to claim 3, wherein
the electrical polarity of (Paa)ₘ-Xaa and Zaa-(Qaa)ₙ is negative,
n is selected from integers of 1 to 20, and
the amino acid present immediately after Zaa is Asp or Glu.

7. The fusion polypeptide according to claim 5 or 6, wherein at least one selected from the group consisting of (Paa)ₘ and (Qaa)ₙ does not comprise a basic amino acid.

8. The fusion polypeptide according to claim 3, wherein
the electrical polarity of (Paa)ₘ-Xaa and Zaa-(Qaa)ₙ is positive,
m is selected from integers of 1 to 20, and
the amino acid present immediately before Xaa is Lys or Arg.

9. The fusion polypeptide according to claim 3, wherein
the electrical polarity of (Paa)ₘ-Xaa and Zaa-(Qaa)ₙ is positive,
n is selected from integers of 1 to 20, and
the amino acid present immediately after Zaa is Lys or Arg.

10. The fusion polypeptide according to claim 8 or 9, wherein at least one selected from the group consisting of (Paa)ₘ and (Qaa)ₙ does not comprise an acidic amino acid.

11. The fusion polypeptide according to any one of claims 1 to 10, wherein a labeling substance is bound to Cys in the Xaa-Cys-Zaa.

12. The fusion polypeptide according to any one of claims 1 to 7, wherein Xaa is Asp, and Zaa is Asp.

13. A method for producing a fusion polypeptide, the method comprising steps of:
producing a fusion polypeptide using a DNA encoding the fusion polypeptide according to any one of claims 1 to 12, and
recovering an expressed fusion polypeptide.

14. The method according to claim 13, further comprising refolding the fusion polypeptide recovered from the host cell.

## Patentansprüche

1. Fusionspolypeptid, umfassend ein Zielpolypeptid und ein Tag-Polypeptid, wobei
das Tag-Polypeptid auf einer N-terminalen Seite und/oder einer C-terminalen Seite des Zielpolypeptids vorliegt und das Tag-Polypeptid eine Struktur umfasst, die durch Xaa-Cys-Zaa dargestellt ist,
i) wobei Xaa für Asp oder Glu und Zaa für Asp oder Glu steht; oder
ii) wobei Xaa für Lys oder Arg und Zaa für Lys oder Arg steht, und
wobei es sich bei dem Zielpolypeptid um einen Antikörper bzw. ein Antikörperfragment handelt und wobei es sich bei dem Antikörperfragment um ein Fab des Antikörpers, ein Fab' des Antikörpers, eine schwere Kette des Antikörpers oder eine leichte Kette des Antikörpers handelt.

2. Fusionspolypeptid nach Anspruch 1, wobei das Tag-Polypeptide eine Struktur aufweist, die durch (Paa)ₘ-Xaa-Cys-Zaa-(Qaa)ₙ dargestellt ist, wobei Paa und Qaa jeweils eine Aminosäure bedeuten, m und n jeweils unabhängig eine Aminosäurezahl bedeuten, die aus ganzen Zahlen von 0 bis 20 ausgewählt ist, und
(Paa)ₘ-Xaa und Zaa-(Qaa)ₙ eine identische elektrische Polarität aufweisen.

3. Fusionspolypeptid nach Anspruch 2, wobei
die elektrische Polarität von (Paa)ₘ-Xaa und Zaa-(Qaa)ₙ negativ ist; oder die elektrische Polarität von (Paa)ₘ-Xaa und Zaa-(Qaa)ₙ positiv ist.

4. Fusionspolypeptid nach einem der Ansprüche 1 bis 3, wobei das Tag-Polypeptid auf der C-terminalen Seite des Zielpolypeptids gebunden ist.

5. Fusionspolypeptid nach Anspruch 3, wobei
die elektrische Polarität von (Paa)ₘ-Xaa und Zaa-(Qaa)ₙ negativ ist,
m aus ganzen Zahlen von 1 bis 20 ausgewählt ist und
die unmittelbar vor Xaa liegende Aminosäure Asp oder Glu ist.

6. Fusionspolypeptid nach Anspruch 3, wobei
die elektrische Polarität von (Paa)ₘ-Xaa und Zaa-(Qaa)ₙ negativ ist,
n aus ganzen Zahlen von 1 bis 20 ausgewählt ist und
die unmittelbar hinter Zaa liegende Aminosäure Asp oder Glu ist.

7. Fusionspolypeptid nach Anspruch 5 oder 6, wobei wenigstens eine ausgewählt aus der Gruppe bestehend aus (Paa)ₘ und (Qaa)ₙ keine basische Aminosäure umfasst.

8. Fusionspolypeptid nach Anspruch 3, wobei
die elektrische Polarität von (Paa)ₘ-Xaa und Zaa-(Qaa)ₙ positiv ist,
m aus ganzen Zahlen von 1 bis 20 ausgewählt ist und
die unmittelbar vor Xaa liegende Aminosäure Lys oder Arg ist.

9. Fusionspolypeptid nach Anspruch 3, wobei
die elektrische Polarität von (Paa)ₘ-Xaa und Zaa-(Qaa)ₙ positiv ist,
n aus ganzen Zahlen von 1 bis 20 ausgewählt ist und
die unmittelbar hinter Zaa liegende Aminosäure Lys oder Arg ist.

10. Fusionspolypeptid nach Anspruch 8 oder 9, wobei wenigstens eine ausgewählt aus der Gruppe bestehend aus (Paa)ₘ und (Qaa)ₙ keine saure Aminosäure umfasst.

11. Fusionspolypeptid nach einem der Ansprüche 1 bis 10, wobei eine Markierungssubstanz an Cys in der Xaa-Cys-Zaa gebunden ist.

12. Fusionspolypeptid nach einem der Ansprüche 1 bis 7, wobei Xaa für Asp und Zaa für Asp steht.

13. Verfahren zur Herstellung eines Fusionspolypeptids, wobei das Verfahren folgende Schritte umfasst:
Herstellen eines Fusionspolypeptids unter Ver-wendung einer das Fusionspolypeptid nach einem der Ansprüche 1 bis 12 codierenden DNA und
Gewinnen eines exprimierten Fusionspolypeptids.

14. Verfahren nach Anspruch 13, ferner umfassend Rückfalten des aus der Wirtszelle gewonnenen Fusionspolypeptids.

## Revendications

1. Polypeptide de fusion comprenant un polypeptide cible et un polypeptide d'étiquette,
le polypeptide d'étiquette étant présent sur un côté N-terminal et/ou un côté C-terminal du polypeptide cible, et le polypeptide d'étiquette comprenant une structure représentée par Xaa-Cys-Zaa,
i) Xaa étant Asp ou Glu, et Zaa étant Asp ou Glu ; ou
ii) Xaa étant Lys ou Arg, et Zaa étant Lys ou Arg, et
le polypeptide cible étant un anticorps ou un fragment d'anticorps, et
le fragment d'anticorps étant un Fab de l'anticorps, un Fab' de l'anticorps, une chaîne lourde de l'anticorps ou une chaîne légère de l'anticorps.

2. Polypeptide de fusion selon la revendication 1, le polypeptide d'étiquette possédant une structure représentée par (Paa)ₘ-Xaa-Cys-Zaa-(Qaa)ₙ,
Paa et Qaa représentant chacun un acide aminé,
m et n représentant chacun indépendamment un nombre d'acides aminés choisi parmi des entiers de 0 à 20, et
(Paa)ₘ-Xaa et Zaa-(Qaa)ₙ possédant une polarité électrique identique.

3. Polypeptide de fusion selon la revendication 2,
la polarité électrique de (Paa)ₘ-Xaa et Zaa-(Qaa)ₙ étant négative ; ou
la polarité électrique de (Paa)ₘ-Xaa et Zaa-(Qaa)ₙ étant positive.

4. Polypeptide de fusion selon l'une quelconque des revendications 1 à 3, le polypeptide d'étiquette étant lié au côté C-terminal du polypeptide cible.

5. Polypeptide de fusion selon la revendication 3,
la polarité électrique de (Paa)ₘ-Xaa et Zaa-(Qaa)ₙ étant négative,
m étant choisi parmi des entiers de 1 à 20, et
l'acide aminé présent immédiatement avant Xaa étant Asp ou Glu.

6. Polypeptide de fusion selon la revendication 3,
la polarité électrique de (Paa)ₘ-Xaa et Zaa-(Qaa)ₙ étant négative,
n étant choisi parmi des entiers de 1 à 20, et
l'acide aminé présent immédiatement après Zaa étant Asp ou Glu.

7. Polypeptide de fusion selon la revendication 5 ou 6, au moins l'un choisi dans le groupe constitué par (Paa)ₘ et (Qaa)ₙ ne comprenant pas un acide aminé basique.

8. Polypeptide de fusion selon la revendication 3,
la polarité électrique de (Paa)ₘ-Xaa et Zaa-(Qaa)ₙ étant positive,
m étant choisi parmi des entiers de 1 à 20, et
l'acide aminé présent immédiatement avant Xaa étant Lys ou Arg.

9. Polypeptide de fusion selon la revendication 3,
la polarité électrique de (Paa)ₘ-Xaa et Zaa-(Qaa)ₙ étant positive,
n étant choisi parmi des entiers de 1 à 20, et
l'acide aminé présent immédiatement après Zaa étant Lys ou Arg.

10. Polypeptide de fusion selon la revendication 8 ou 9, au moins l'un choisi dans le groupe constitué par (Paa)ₘ et (Qaa)ₙ ne comprenant pas un acide aminé acide.

11. Polypeptide de fusion selon l'une quelconque des revendications 1 à 10, une substance de marquage étant liée à Cys dans le Xaa-Cys-Zaa.

12. Polypeptide de fusion selon l'une quelconque des revendications 1 à 7, Xaa étant Asp, et Zaa étant Asp.

13. Procédé pour la production d'un polypeptide de fusion, le procédé comprenant les étapes de :
production d'un polypeptide de fusion en utilisant un ADN codant pour le polypeptide de fusion selon l'une quelconque des revendications 1 à 12, et
récupération d'un polypeptide de fusion exprimé.

14. Procédé selon la revendication 13, comprenant en outre le repliement du polypeptide de fusion récupéré de la cellule hôte.
